**Europäisches Patentamt**

(19) **European Patent Office**

**Office européen des brevets**

(11) Veröffentlichungsnummer: **0 036 152**
**B1**

(12) ## EUROPÄISCHE PATENTSCHRIFT

(45) Veröffentlichungstag der Patentschrift:
**18.05.83**

(21) Anmeldenummer: **81101667.4**

(22) Anmeldetag: **07.03.81**

(51) Int. Cl.³: **C 07 C 89/02**, C 07 C 91/04,
C 07 C 85/06, C 07 C 87/14,
B 01 J 19/02

(54) **Verfahren zur Herstellung bzw. Umsetzung von Alkanolaminen.**

(30) Priorität: **15.03.80 DE 3010105**
**29.11.80 DE 3045148**

(43) Veröffentlichungstag der Anmeldung:
**23.09.81 Patentblatt 81/38**

(45) Bekanntmachung des Hinweises auf die Patenterteilung:
**18.05.83 Patentblatt 83/20**

(84) Benannte Vertragsstaaten:
**AT BE DE FR GB IT LU NL SE**

(56) Entgegenhaltungen:
**DE-A-1 543 692**
**DE-A-2 358 355**
**DE-A-2 624 109**
**DE-A-2 810 135**

(73) Patentinhaber: **BASF Aktiengesellschaft,
Carl-Bosch-Strasse 38, D-6700 Ludwigshafen (DE)**

(72) Erfinder: **Boettger, Guenter, Dr., Langer Wingert 16 b,
D-6702 Bad Duerkheim (DE)**
Erfinder: **Hammer, Hans, Dipl.-Ing., Waldlichtung 42,
D-6800 Mannheim (DE)**
Erfinder: **Hertel, Otto, Dr., Koenigsbacherstrasse 68,
D-6700 Ludwigshafen (DE)**
Erfinder: **Jeschek, Gerhard, Dr., Im Zaunruecken 14,
D-6718 Gruenstadt (DE)**
Erfinder: **Mueller, Herbert, Dr., Carostrasse 53,
D-6710 Frankenthal (DE)**
Erfinder: **Scharf, Emil, Dr., Mohnstrasse 51,
D-6700 Ludwigshafen (DE)**
Erfinder: **Schoenleben, Willibald, Dr.,
Blumenthalstrasse 1, D-6900 Heidelberg (DE)**

## Verfahren zur Herstellung bzw. Umsetzung von Alkanolaminen

Die Erfindung betrifft ein Verfahren zur Herstellung von ungefärbten, auch bei der Lagerung nicht verfärbenden Alkanolaminen aus Ammoniak bzw. umsetzungsfähigen Aminen und Alkylenoxid, und/oder deren weitere Umsetzung mit Ammoniak zu Alkylendiaminen bzw. -polyaminen, bzw. deren Reingewinnung.

Die an sich farblosen Alkanolamine, insbesondere Di- und Triethanolamin sind als technische Produkte in der Regel durch unbestimmte Beimengungen gelblich bis bräunlich verfärbt. Bei der Lagerung vertieft sich der Farbton gewöhnlich und entwertet so die Alkanolamine für den typischen Gebrauchszweck, z.B. im Bereich der Herstellung von Kosmetika, Pharma-Artikeln und tensidhaltigen anderen Hilfsmitteln.

Diese Erscheinung wird noch lästiger dadurch, dass bei der Salzbildung der Amine ein Farbumschlag zu braunen oder blauen, i.a. noch dichteren Tönen eintritt. Die einmal aufgetretene Verfärbung lässt sich übrigens kaum mehr beseitigen, zumal die Destillation von Alkanolaminen bekanntlich hohe Temperaturen und beträchtlichen Trennaufwand erfordert.

Das gleiche Problem tritt auch auf bei der Weiterverarbeitung der Alkanolamine, d.h. der Herstellung oder Verarbeitung von aliphatischen oder cylcoaliphatischen Diaminen und Polyaminen, die gegebenenfalls auch alkoholische OH-Gruppen enthalten können. Als Beispiele seien die Umsetzungsprodukte von Alkanolaminen mit Ammoniak, d.h. Ethylendiamin bzw. Propylendiamin und die bei der Diamin-Synthese anfallenden Nebenprodukte, also z.B. Diethylentriamin, Triethylentetramin, Piperazin, N-(2-Aminoethyl-)piperazin, N-(2-Hydroxyethyl-)piperazin, N-(2-Aminoethyl-)ethanolamin genannt.

Einen gewissen Hinweis für die Herkunft der Beimengungen liefert die Tatsache, dass die Herstellung bzw. Umsetzung der Alkanolamine häufig von starker Korrosion der verwendeten Anlagenteile begleitet ist. Zur technischen Herstellung und Verarbeitung der genannten Verbindungen werden stets Apparate aus den gängigen Chrom-Nickel-Stahlsorten (nichtrostenden Stahlsorten) verwendet, wie z.B. Nr.1.4541, 1.450 oder 1.4571. Denn diese Werkstoffe bewirken bei geringer Neigung zur Spannungsrisskorrosion einen guten Wärmeübergang und erlauben bei erhöhten Temperaturen die Anwendung von Überdruck oder von vermindertem Druck, wie dies bei der Synthese und destillativen Aufarbeitung erforderlich ist. An sich bekannte andere sog. nichtrostende Stähle werden in der Apparatetechnik deswegen nicht verwendet, weil sie z.B. nicht schweissbar sind oder bei Druckbelastung zur Spannungsrisskorrosion neigen. Obwohl die in der chemischen Technik verwendeten druckfesten, nichtrostenden Stähle, die praktisch ausschliesslich den austenitischen Chrom-Nikkelstählen zuzurechnen sind (vgl. hierzu die Einteilung nach L. Piatti «Werkstoffe der chemischen Technik», Reihe «Grundlagen der Chemischen Technik», Band 3, S. 264 bis 268), als gut korrosionsfest gegenüber Aminen gelten, treten bei Berührung mit den oben genannten Verbindungen stellenweise erhebliche Korrosionserscheinungen auf. Besonders Einbauten in Kolonnen und Reaktoren sind gefährdet. Andererseits enthalten z.B. selbst tief verfärbte Alkanolamine keine signifikanten Mengen an Schwermetallen.

Dem Schrifttum sind keine Hinweise auf die Herkunft der Verfärbungen zu entnehmen.

Da die Verwendung keramischer oder gläserner Bauteile wegen der notwendigen Drucke und der stark alkalischen Reaktionsgemische praktisch ausscheidet, hat man bisher versucht, durch Zusätze die Störungen günstig zu beeinflussen; allerdings konnten bisher und in gewissem Rahmen nach der DE-AS 28 10 185 nur die Verfärbungen durch Zusätze von phosphoriger Säure o.ä. beherrscht werden. Die Korrosion liess sich damit nicht ausreichend zurückdrängen.

Aufgabe der Erfindung ist die Vermeidung der Verfärbung von Reaktionsprodukten der Umsetzungsreihe: Ammoniak → Alkanolamine → Alkylendiamine bzw. -polyamine und die Bekämpfung von Korrosionserscheinungen.

Es wurde nun gefunden, dass sich Korrosion und Verfärbungen bei den besagten Verfahren vermeiden lassen, wenn man anstelle üblicher nickellegierter nichtrostender Stähle, d.h. Stählen, deren Korrosionsbeständigkeit durch Zulegieren von Nickel bewirkt wird, im wesentlichen nickelfreie Stähle als Apparatewerkstoff verwendet; als «im wesentlichen nickelfrei» wird hier ein Edelstahl mit weniger als 0,5% Nickel bezeichnet.

Vielfach gelingt eine deutliche Verbesserung bei vorhandenen Anlagen schon dadurch, dass man einzelne Bauteile, an denen Korrosion aufgetreten ist, durch solche aus nickelarmen oder nickelfreiem Material ersetzt. Besonders empfehlenswert ist die Verwendung nickelfreier Materialien bei Einbauten wie Füllkörpern, Wärmetauscherrohren und generell solchen Teilen, die hoher thermischer oder mechanischer Belastung (Strömungsgeschwindigkeit) ausgesetzt sind.

Bevorzugt werden sehr kohlenstoffarme (0,025%) ferritische Stähle, die z.B. 17 bis 19% Chrom, 1,75 bis 2,5% Molybdän, bis 0,5% Mangan, bis 1% Silicium und bis 0,025% Stickstoff enthalten.

Dieses Ergebnis ist deshalb überraschend, weil die übliche Korrosionsvorprüfung im Labormassstab für die Auswahl von geeignetem Apparatematerial im Falle der Alkanolamine den Einsatz von üblichen nickelhaltigen Edelstählen durchaus empfiehlt und nickelfreie Stähle ausschliesst. Offensichtlich verhält sich eine Anlage im praktischen Betrieb anders als nur begrenzte Zeit betriebene Modelle. In Anbetracht der Ergebnisse von Korrosionsprüfungen bedurfte es also der Überwindung eines gewissen Vorurteils, um zur Erfindung zu gelangen. Auch andere Hinweise, wie z.B. bei Kirk-Othmer, Encyclopedia of Chemical Technology, 3. Auflage, Band 1, S. 955, wo zur Erzielung unverfärbter Alkanolamine die Anwendung von rostfreiem Stahl empfohlen wird und in Corrosion Data Survey/Metals Section (Hrsgg. v. N.E. Hamner, Nat. Ass of Corrosion Engs., Houston, Tx., USA), S. 184, das für Triethanolamin nur geringe Korrosion selbst bei heissen wässrigen Lösungen angibt, sprechen gegen einen Zusammenhang zwischen der Verwendung von nichtrostendem Stahl und Korrosion durch Alkanolamine und geben erst recht keinen Hinweis darauf, wo es auf eine spezielle Materialauswahl ankommt.

Beispiel für im wesentlichen nickelfreien Edelstahl ist Werkstoff Nr. 1.4521 (1803 MoT). Die Apparate können vollständig oder teilweise, z.B. an bislang korrosionsgefährdeten Stellen aus derartigem Material bestehen, oder es können Auskleidungen benutzt werden. Die Verwendung nickelfreier Stähle ist besonders bei Apparateteilen zu empfehlen, die hoher thermischer und mechanischer Belastung ausgesetzt sind, wie z.B. Reaktoren, Verdampfer, Wäremaustauscher, Füllkörper und Destillierböden in Destillationskolonnen.

Die Erfindung lässt sich überall da anwenden, wo Alkanolamine und inbesondere Ethanolamine als Ausgangsstoffe oder Produkte in erhitzten Reaktionsgemischen auftreten, also bei der eigentlichen Herstellung ebenso wie bei der Auftrennung der Alkanolamine (Destillation) und bei weiterführenden Umsetzungen sowie bei Verfahren, bei denen Alkanolamine im wesentlichen physikalische Wirkungen entfalten wie bei der Gaswäsche ($CO_2$-Wäsche). Die zur Herstellung von Ethanolaminen geeigneten Vorrichtungen sind vielfach auch zu deren Weiterverarbeitung und zur Herstellung und Weiterverarbeitung höherer Alkanolamine (z.B. der Propanolamine) geeignet, besonders bei Verfahren, die unter Druck ausgeübt werden.

Beispiel 1

In einer technischen Anlage, deren Reaktor aus gewöhnlichem Stahl und deren Aufarbeitungsteil aus teils gewöhnlichem Stahl, teils üblichem Chrom-Nickel-Stahl (V2A) besteht, wird wasserhaltiger Ammoniak mit Ethylenoxid bei 120 bis 140°C und einem Druck von 80 bar umgesetzt.

Im Destillationsteil treten Temperaturen bis 180°C auf, während der Druck stufenweise von 20 bar auf 0,6 mbar zurückgenommen wird.

Nach dem Destillationsergebnis hat die Umsetzung zu je 60 bzw. 30 bzw. 10% Mono- bzw. Di- bzw. Triethanolamin (MEA, DEA, TEA) geführt.

Der Materialverlust beträgt jährlich zwischen 1 mm (Normalstahl) und 0,5 mm (Edelstahl).

Die Anlage wurde durch Ersatz von etwa 1000 m² Füllkörpern und 400 m² Wärmeaustauschern (Rohrbündel) aus V2A-Stahl durch solchen aus Cr-Stahl 1803 Mo, T umgerüstet.

Zusätzlich werden die aus Normalstahl bestehenden Verbindungsrohre der Verdampfer im Ammoniakdestillationssystem durch Rohre aus dem schwach Ni-haltigen Stahl 1.4462 ersetzt.

Die Betriebsdaten wurden nicht verändert. Bei den ersetzten Teilen wurde nach einem Beobachtungszeitraum von 1 Jahr keinerlei Korrosionsabtrag festgestellt.

Die Farbzahlen der Produkte vor und nach Einbau der neuen Anlagenteile sind in der nachfolgenden Tabelle 1 wiedergegeben (Farbzahl nach APHA).

Die gleichen Produkte wurden ausserdem mit Essigsäure umgesetzt und die Farbzahlen ebenso bestimmt (Tabelle 2, APHA).

Tabelle 1 – Farbzahlen nach APHA

| | vor Einbau | | nach Einbau | |
|---|---|---|---|---|
| | frische Produkte | 3 Mon. gelagerte Produkte | frisch | 3 Mon. gelagert |
| Prod. | | | | |
| MEA | 11,1 | 26,8 | 1,5 | 3,1 |
| DEA | 15,8 | 105,5 | 2,5 | 4,8 |
| TEA | 82,8 | ca. 350 | 5,7 | 12,5 |

Tabelle 2 – Farbzahlen nach APHA

| | | | | |
|---|---|---|---|---|
| MEA | 16,3 | 32,7 | 3,1 | 5,6 |
| DEA | 32,6 | 78,1 | 4,2 | 7,1 |
| TEA | tiefblau (–) | schwarzblau (–) | 20,3 | 25 |

Beispiel 2

Jeweils 300 g eines technischen Polyamins wurden in Gegenwart von 30 g Stahlspänen bestimmter Sorte unter Rühren 4 Stunden lang erhitzt. Nach Abkühlung und Filtration wurde die Qualität der Produkte geprüft. Die Ergebnisse sind folgender Tabelle zu entnehmen, wobei sich die Überlegenheit des nickelfreien Stahls zeigt.

| Stoff | Stahl | Temperatur °C | Farbton nach Jodskala | Bestimmung des Destillations- rückstands |
|-------|-------|---------------|------------------------|-------------------------------------------|
| N-(2-Amino- ethyl-)ethanol- amin | 1.4550 (V2A) (10,5% Ni) | 238 | 120 | 0,80% |
| | 1.4521 1802 MoT; (prakt. Ni-frei) | 238 | 10 | 0,35% |
| N-(2-Hydroxy- ethyl-) piperazin | 1.4550 | 241 | 15 | 0,40% |
| | 1.4521 | 241 | 7 | 0,25% |

Beispiel 3

Bei der Umsetzung von Ethanolamin mit Ammoniak zu Ethylendiamin erhält man ein Produktgemisch. Aus diesem werden das niedrigsiedende Ethylendiamin und Piperazin abdestilliert. Die verbleibenden Hochsieder, die grösstenteils aus Ethanolamin, Diethylentriamin und N-(2-Aminoethyl-)ethanolamin bestehen, werden durch Destillation in Stahlapparaturen weiter getrennt. Nickelfreier Stahl ist hierfür besser geeignet als der übliche nickelhaltige Stahl, wie folgender Versuch zeigt.

Je 300 g eines Modell-Gemischs aus Diethylentriamin, N-(2-Aminoethyl-)ethanolamin und Ethanolamin wurden mit nickelhaltigen und nickelfreien Stahlspänen 24 Stunden lang bei 170°C gerührt. Danach wurden folgende Produkt-Qualitäten festgestellt:

| Stahlsorte | Farbton nach Jodskala | Bestimmung des Destillations- rückstands |
|------------|------------------------|-------------------------------------------|
| 1.4541 (V2A) (10,5% Ni) | 15 | 1,5% |
| 1.4521 (prakt. Ni-frei) | 7 | 0,2% |

**Patentansprüche**

1. Verfahren zur Herstellung von Alkanolaminen und/oder Alkylendiaminen durch thermische Umsetzung von Alkylenoxid bzw. Alkanolaminen mit Ammoniak oder umsetzungsfähigen Aminen und/oder thermische Trennung der gebildeten Reaktionsgemische in stählernen Apparaturen, dadurch gekennzeichnet, dass man nichtrostenden Stahl mit einem Gehalt von weniger als 0,5% Nickel als Werkstoff für solche Stellen, die erfahrungsgemäss der Korrosion unterliegen oder für alle Apparateteile verwendet.

2. Verfahren nach Anspruch 1, dadurch gekennzeichnet, dass man korrosionsbeständigen Stahl mit weniger als 0,025% Kohlenstoff verwendet.

3. Verfahren nach Anspruch 1, dadurch gekennzeichnet, dass als Alkylenoxid Ethylenoxid oder Propylenoxid eingesetzt werden.

**Claims**

1. A process for preparing alkanolamines and/or alkylenediamines by thermally reacting alkylene oxides or alkanolamines with ammonia or reactive amines and/or thermally separating the resulting reaction mixtures in steel apparatus, wherein stainless steel containing less than 0.5% of nickel is used as material of construction at those points which normally suffer corrosion, or for all parts of the apparatus.

2. A process as claimed in claim 1, wherein a corrosion-resistant steel containing less than 0.025% of carbon is used.

3. A process as claimed in claim 1, wherein the alkylene oxide used is ethylene oxide or propylene oxide.

**Revendications**

1. Procédé de préparation d'alcanol-amines et (ou) d'alcoylène-diamines par réaction thermique d'un oxyde d'alcoylène ou d'alcanolamines avec l'ammoniac ou des amines réactives et(ou) séparation thermique des mélanges réactionnels formés dans un appareillage en acier, caractérisé en ce que l'on emploie comme matériau pour les parties généralement sujettes à la corrosion ou pour l'appareillage complet un acier inoxydable avec une teneur en nickel inférieure à 0,5%.

2. Procédé suivant la revendication 1, caractérisé en ce que l'on emploie un acier résistant à la corrosion avec une teneur en carbone inférieure à 0,025%.

3. Procédé suivant la revendication 1, caractérisé en ce que l'oxyde d'alcoylène mis en œuvre est l'oxyde d'éthylène ou l'oxyde de propylène.